# EUROPÄISCHE PATENTSCHRIFT

(11) **EP 0 868 463 B1**
(45) Veröffentlichungstag und Bekanntmachung des Hinweises auf die Patenterteilung: **07.07.1999**
(21) Anmeldenummer: 96943965.2
(22) Anmeldetag: 16.12.1996
(51) Int. Cl.: C08G 18/80, C08G 18/78, C09D 175/04

(54) **VERBINDUNGEN MIT ISOCYANATGRUPPEN UND VERKAPPTEN GEGENÜBER ISOCYANATEN REAKTIVEN GRUPPEN**
COMPOUNDS WITH ISOCYANATE GROUPS AND MASKED GROUPS REACTIVE IN RELATION TO ISOCYANATES
COMPOSE A GROUPES ISOCYANATE ET A GROUPES MASQUES REACTIFS VIS-A-VIS DES ISOCYANATES

(30) Priorität: 21.12.1995 DE 19547974
(43) Veröffentlichungstag der Anmeldung: 07.10.1998
(73) Patentinhaber: BASF AKTIENGESELLSCHAFT, 67056 Ludwigshafen (DE)
(72) Erfinder: BRUCHMANN, Bernd, D-67251 Freinsheim (DE); RENZ, Hans, D-67149 Meckenheim (DE); MOHRHARDT, Günter, D-67346 Speyer (DE)
(86) Internationale Anmeldenummer: EP9605634
(87) Internationale Veröffentlichungsnummer: WO9723536

(56) Entgegenhaltungen:
- EP-A- 0 585 835
- EP-A- 0 682 012
- DE-A- 19 524 046
- US-A- 4 002 601

## Beschreibung

Die Erfindung betrifft Verbindungen mit Isocyanatgruppen und verkappten gegenüber Isocyanaten reaktiven Gruppen der allgemeinen Formel I. 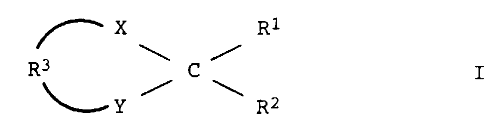 in denen R¹, R², R³, X und Y die folgende Bedeutung haben:
- R¹, R²: Wasserstoff, C₁- bis C₁₀-Alkyl, C₆- bis C₁₀-Aryl, C₇- bis C₁₀-Aralkyl, oder R¹ und R² bilden gemeinsam C₃- bis C₁₀-Alkandiyl,
- X, Y: -O-, -S-, 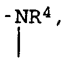 wobei R⁴ Wasserstoff eine C₁- bis C₂₀-Alkylgruppe, die gegebenenfalls durch Sauerstoffatome in Etherfunktion unterbrochen ist, eine C₆- bis C₁₀-Arylgruppe oder eine C₇- bis C₁₀-Aralkylgruppe bedeutet,
- R³: eine C₁- bis C₁₀-Alkandiylgruppe, die zusammen mit der Einheit -X-CR¹R²-Y- einen 4 bis 7gliedrigen Ring bildet,
wobei entweder bei der Einheit R³ eines der Wasserstoffatome oder bei der Einheit 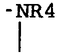 der Rest R⁴ durch eine
Allophanat-Gruppe R^{Ia} 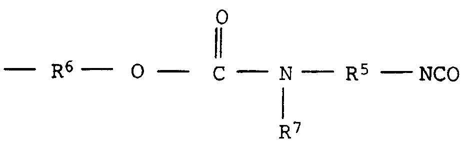 in der
- R⁵: eine divalente aliphatische, alicyclische, araliphatische oder aromatische C₂- bis C₂₀-Kohlenwasserstoffeinheit und
- R⁶: eine Einfachbindung oder eine divalente aliphatische, alicyclische, araliphatische oder aromatische C₁- bis C₂₀-Kohlenwasserstoffeinheit oder eine Mono- oder Poly-(C₂- bis C₄-Alkylenoxid)-Einheit und
- R⁷: ein Carbamoylrest 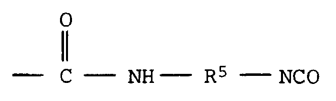 bedeutet
oder eine Biuret-Gruppe R^{Ib} 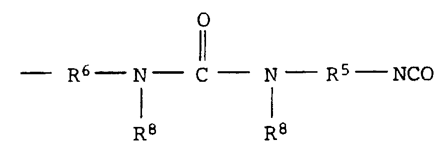 in der einer der Reste R⁸ Wasserstoff und der andere Rest die gleiche Bedeutung wie R⁷ hat
oder eine Biuret-Gruppe R^{Ic} 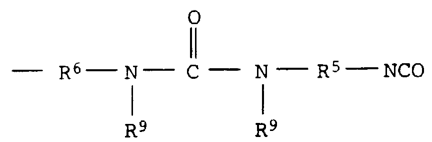 in der einer der Reste R⁹ die gleiche Bedeutung wie R⁷ und der andere Rest die gleiche Bedeutung wie R¹ hat
oder eine Thioallophanat-Gruppe R^{Id} 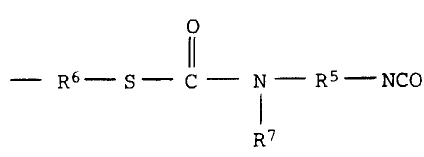 ersetzt ist.

Weiterhin betrifft die Erfindung vernetzende Polyurethanbeschichtungsmassen, die Verbindungen der Formel I enthalten sowie Beschichtungsverfahren, bei denen diese vernetzenden Polyurethanbeschichtungsmassen Verwendung finden.

Vernetzende Polyurethanbeschichtungsmassen sind z.B. in Form von 2-K-Lacken allgemein bekannt (vgl. Kunststoff Handbuch, Band 7, Polyurethan, 2. Auflage, 1983, Carl Hanser Verlag München Wien, S. 540 bis 561). Diese Zweikomponentensysteme enthalten als Bindemittel ein Polyol und als Vernetzerkomponente eine Verbindung mit mehreren freien Isocyanatgruppen.

Weil sich nach der Vermischung der beiden Komponenten ein hochmolekulares Netzwerk ausbildet, dürfen die beiden Komponenten erst unmittelbar vor der Applikation auf das zu beschichtende Werkstück vermischt werden. Hieraus ergibt sich für den Verarbeiter dieser Systeme das Erfordernis, die einzelnen Arbeitsschritte beim Beschichten zeitlich gut aufeinander abstimmen zu müssen, da er nach dem Vermischen der Komponenten eine definierte Lackmenge innerhalb einer bestimmten Zeit verarbeiten muß.

Dieser Aufwand bei der Verarbeitung dieser Systeme wird vom Verarbeiter in Kauf genommen, denn die daraus hergestellten Beschichtungen sind solchen aus den allgemein bekannten einkomponentigen nicht-vernetzenden Systemen, die prinzipiell beliebig lange lagerbar sind, deutlich überlegen.

Dies betrifft insbesondere Gebrauchseigenschaften wie
- Unempfindlichkeit gegenüber mechanischer Beanspruchung wie Zug, Dehnung, Schlägen oder Abrieb
- Resistenz gegenüber Feuchtigkeit (z.B. in Form von Wasserdampf) und verdünnten Chemikalien
- Beständigkeit gegenüber Umwelteinflüssen wie Temperaturschwankungen und UV-Strahlung
- hoher Glanz der beschichteten Oberflächen.

Damit Lacke problemlos mit üblichen Verfahren, z.B. durch Aufsprühen auf die zu beschichtende Oberfläche, aufgetragen werden können, sollen die Lacke eine begrenzte Viskosität aufweisen. Lacke auf Basis von Zweikomponenten-Systemen enthalten deshalb üblicherweise Lösungsmittel. Der Lösungsmittelgehalt dieser Lacke bereitet jedoch Probleme, da die Verarbeiter der Lacke technisch aufwendige Maßnahmen ergreifen müssen, um zu vermeiden, daß die Lösungsmittel, die beim Auftrag und Trocknen der Lacke freigesetzt werden, nicht in die Atmosphäre gelangen. Es wurde deshalb vielfach versucht, einen Ausweg aus diesem Zielkonflikt zu finden und Zweikomponenten-Systeme bereitzustellen, die eine niedrige Viskosität, gleichzeitig einen hohen Festkörperanteil und daneben auch ein hohes Eigenschaftsprofil aufweisen, was die genannten Gebrauchseigenschaften betrifft.

In der EP-A-0585835 sind Zweikomponenten-Systeme beschrieben, wobei es sich bei der Isocyanat-Komponente um trimerisierte Diisocyanate mit Isocyanuratgruppen handelt, bei denen ein Teil der verbleibenden Isocyanatgruppen mit einem einwertigen Alkohol zu Urethangruppen umgesetzt sind.

Polyisocyanate mit Isocyanuratgruppen und Allophanatgruppen aus einem Diisocyanat und einem Polyestermonoalkohol sind in der DE-A-O 15 155 beschrieben. Diese Polyisocyanate können als Härter in Zweikomponenten-Polyurethanlacken verwendet werden.

Zur Erniedrigung der Viskosität der Zweikomponentensysteme wurden weiterhin sog. Reaktivverdünner entwickelt. Diese Verbindungen beeinflussen die Viskosität der Zweikomponenten-Systeme ähnlich wie ein Lösungsmittel. Im Unterschied zu Lösungsmitteln reagieren die Reaktivverdünner jedoch bei der Trocknung bzw. Härtung der als Lack eingesetzten Zweikomponenten-Systeme mit den anderen Bindemittelkomponenten unter Ausbildung eines hochmolekularen Netzwerkes.

Das Dokument US-A-4,002,601 offenbart ein Verfahren zur Herstellung von Urethanen, die Oxazolidin- und gegebenfalls Isocyanat-Gruppen enthalten, dadurch gekennzeichnet, daß N-Hydroxyalkyl-Oxazolidine mit organischen Polyisocyanaten bei einem OH/NCO Verhältnis von 1:1 bis 1:6 umgesetzt werden.

Die deutsche Patentanmeldung mit dem Aktenzeichen P 19524046.4 beschreibt einen Reaktivverdünner, bei dem es sich um eine Verbindung mit einer Isocyanat-, einer Urethan-, Thiourethan- oder Harnstoffgruppe und 2 verkappten, mit Isocyanat reaktionsfähigen Gruppen handelt. Diese Verbindungen enthalten jedoch weder eine Allophanat- noch eine Biuretgruppe.

Obwohl mit den vorbekannten Zweikomponenten-Systemen bereits gewisse Fortschritte, was die Reduzierung des Lösungsmittelgehaltes betrifft, erzielt werden konnten, besteht in dieser Hinsicht nach wie vor ein Verbesserungsbedarf.

Aufgabe der vorliegenden Erfindung waren deshalb Zweikomponenten-Systeme, welche bei hohem Feststoffgehalt eine niedrige Viskosität aufweisen, die sich zu Beschichtungen mit einem guten Eigenschaftsprofil verarbeiten lassen sowie Komponenten hierfür. Aufgabe waren weiterhin vernetzende Einkomponentensysteme, welche bei hohem Feststoffgehalt eine niedrige Viskosität aufweisen, die lagerstabil sind und sich zu Beschichtungen verarbeiten lassen, die denen aus Zweikomponenten-Systemen zumindest gleichwertig sind, sowie Komponenten hierfür.

Demgemäß wurden die eingangs definierten Verbindungen (I) mit Isocyanatgruppen und verkappten, gegenüber Isocyanatgruppen reaktiven Gruppen gefunden. Weiterhin wurden vernetzende Einkomponenten- und Zweikomponenten-Polyurethanbeschichtungsmittel gefunden, die diese Verbindungen (I) enthalten.

Unter den erfindungsgemäßen Verbindungen der Formel I sind solche bevorzugt, bei denen R¹, R², R³, X und Y die folgende Bedeutung haben:

Als Reste R¹ und R² kommen insbesondere Wasserstoff, C₁- bis C₆-Alkyl, besonders Methyl, Ethyl und iso-Propyl in Betracht. Unter den C₃- bis C₁₀-Alkandiylgruppen, die R¹ und R² gemeinsam bilden können, sind Cyclopentyl und Cyclohexyl bevorzugt.

Bevorzugte Reste R⁴ in der Gruppe 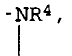 die für X und/oder Y stehen kann, sind C₁- bis C₆-Alkylgruppen, z.B. Methyl, Ethyl, n-Propyl oder n-Butyl.

Als Gruppen R³ kommen vor allem C₂- bis C₁₀-Alkandiylgruppen in Betracht, die zusammen mit der Einheit -X-CR¹R²-Y- einen 5- oder 6-gliedrigen Ring bilden.

In den Verbindungen der Formel I ist zwingend entweder ein Wasserstoffatom der Einheit R³ oder der Rest R⁴ durch eine Allophanatgruppe R^{Ia}, eine Biuret-Gruppe R^{Ib}, eine Biuret-Gruppe R^{Ic} oder eine Thioallophanat-Gruppe R^{Id} substituiert. Die Verbindungen der Formel I tragen also eine einzige Gruppe, ausgewählt aus der Menge der Gruppen R^{Ia}, R^{Ib}, R^{Ic} und R^{Id}. Die Gruppe, bei der es sich um eine Gruppe R^{Ia}, R^{Ib}, R^{Ic} und R^{Id} handeln kann, wird im folgenden Text als R^{I} bezeichnet.

Besonders bevorzugt sind Dioxolane der Formel I.1 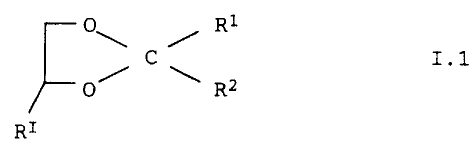 Dioxane der Formel I.2 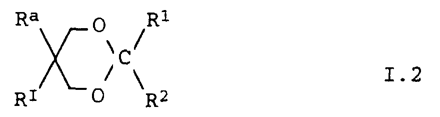 wobei R^{a} ein Wasserstoffatom oder ein C₁- bis C₁₀-Alkylrest bedeutet,
oder Oxazolidinderivate der Formel I.3.

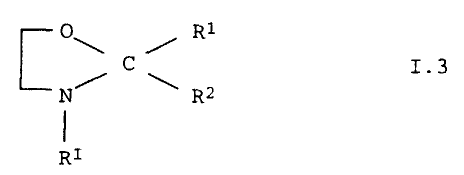

Bei den Einheiten R⁵, die Bestandteil der Reste R^{I} sind, handelt es sich bevorzugt um Einheiten, die sich von üblichen Diisocyanaten (vgl. Kunststoff Handbuch, Band 7, Polyurethan, 2. Auflage, 1983, Carl Hanser Verlag München Wien, Kapitel 2.2.1) durch Abstraktion der beiden Isocyanatgruppen ableiten.

Beispiele für übliche Diisocyanate sind aliphatische Diisocyanate wie Tetramethylendiisocyanat, Hexamethylendiisocyanat(1,6-Diisocyanatohexan), Octamethylendiisocyanat, Decamethylendiisocyanat, Dodecamethylendiisocyanat, Tetradecamethylendiisocyanat, Derivate des Lysindiisocyanates, Tetramethylxylylendiisocyanat, Trimethylhexandiisocyanat oder Tetramethylhexandiisocyanat, cycloaliphatische Diisocyanate wie 1,4-, 1,3- oder 1,2-Diisocyanatocyclohexan, 4,4'-Di(isocyanatocyclohexyl)methan, 1-Isocyanato-3,3,5-trimethyl-5-(isocyanatomethyl)cyclohexan(Isophorondiisocyanat) oder 2,4-, oder 2,6-Diisocyanato-1-methylcyclohexan sowie aromatische Diisocyanate wie 2,4- oder 2,6-Toluylendiisocyanat, p-Xylylendiisocyanat, 2,4'- oder 4,4'-Diisocyanatodiphenylmethan, 1,3- oder 1,4-Phenylendiisocyanat, 1-Chlor-2,4-phenylendiisocyanat, 1,5-Naphthylendiisocyanat, Diphenylen-4,4'-diisocyanat, 4,4'-Diisocyanato-3,3'-dimethyldiphenyl, 3-Methyldiphenylmethan-4,4'-diisocyanat oder Diphenylether-4,4'-diisocyanat. Es können auch Gemische der genannten Diisocyanate vorliegen. Bevorzugt sind Hexamethylendiisocyanat und Isophorondiisocyanat, sowie 2,4- und 2,6-Toluylendiisocyanat und 2,4'- und 4,4'-Diphenylmethandiisocyanat.

Bei den Einheiten R⁶ sind verzweigte und unverzweigte C₁- bis C₄-Alkandiylgruppen bevorzugt. Besonders bevorzugt ist Methylen oder Ethylen. Weiterhin sind als Einheiten R⁶ Mono- oder Poly(C₂-bis C₄-Alkylenoxid)-Einheiten der Formel 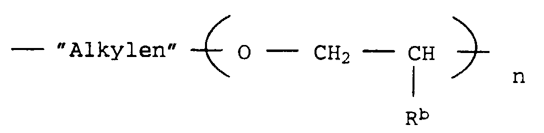 wobei "Alkylen" eine verzweigte oder unverzweigte C₁- bis C₄-Alkandiylgruppe ist, n eine Zahl von 1 bis 20 und R^{b} unabhängig voneinander Methyl, Ethyl oder Wasserstoff bedeutet.

Dabei kommen sowohl homo- als auch copolymere Einheiten abgeleitet von Ethylenoxid, Propylenoxid oder Butylenoxid in betracht.

Die erfindungsgemäßen Verbindungen der Formel I werden als B-Komponente in Zweikomponenten-Polyurethanbeschichtungsmitteln üblicherweise in Form von Mischungen (B) eingesetzt, die enthalten
a) Verbindungen der allgemeinen Formel I,
b) übliche Isocyanate mit im Mittel wenigstens 2 Isocyanat-Gruppen (Verbindungen II)
c) Verbindungen der Formel III 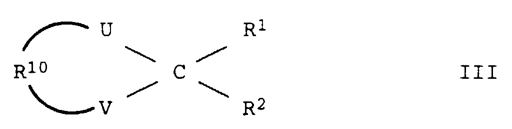 in der R¹, R², R¹⁰, U und V die folgende Bedeutung haben:
   - R¹, R²: die bei Formel I angegebene Bedeutung
   - U, V: -O-, -S-, 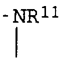 wobei R¹¹ Wasserstoff, eine C₁- bis C₁₀-Alkylgruppe, die gegebenenfalls durch Sauerstoffatome in Etherfunktion unterbrochen ist, eine C₆- bis C₁₀-Arylgruppe oder eine C₇- bis C₁₀-Aralkylgruppe,
   - R¹⁰: eine C₁- bis C₁₀-Alkandiylgruppe, die zusammen mit -U-CR¹R²-V- einen 4- bis 7-gliedrigen Ring bildet, wobei entweder bei der Einheit R¹⁰ eines der Wasserstoffatome oder bei der Einheit 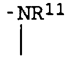 der Rest R¹¹ durch eine Urethan-Gruppe R^{IIIa} 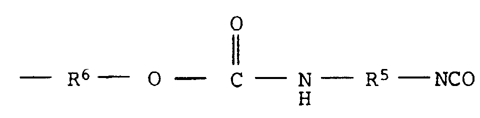 oder eine Harnstoff-Gruppe R^{IIIb} 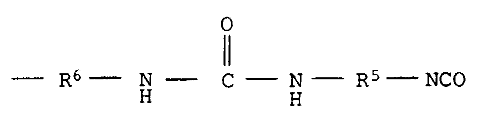 oder eine Harnstoff-Gruppe R^{IIIc} 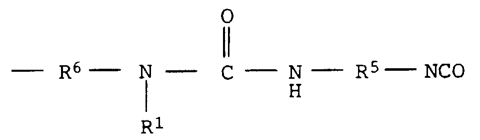 oder eine Thiourethan-Gruppe R^{IIId} 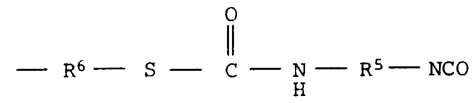 wobei R⁵ und R⁶ die in Formel I angegebene Bedeutung haben,
   substituiert ist.

Als übliche Isocyanate mit im Mittel wenigstens 2 Isocyanat-gruppen (Verbindungen II) eignen sich beispielsweise Triisocyanate wie 2,4,6-Triisocyanatotoluol, Triphenylmethantriisocyanat oder 2,4,4'-Triisocyanatodiphenylether oder die Gemische aus Di-, Tri- und höheren Polyisocyanaten, die durch Phosgenierung von entsprechenden Anilin/Formaldehyd-Kondensaten erhalten werden und Methylenbrücken aufweisende Polyphenylpolyisocyanate darstellen.

Von besonderem Interesse sind als Verbindungen II übliche aliphatische höherfunktionelle Polyisocyanate der folgenden Gruppen:
(a) Isocyanuratgruppen aufweisende Polyisocyanate von aliphatischen, cycloaliphatischen, aromatischen und/oder araliphatischen Diisocyanaten. Besonders bevorzugt sind hierbei die entsprechenden Isocyanato-Isocyanurate auf Basis von Hexamethylendiisocyanat und Isophorondiisocyanat. Bei den vorliegenden Isocyanuraten handelt es sich insbesondere um einfache Tris-isocyanatoalkyl- bzw. Tris-isocyanatocycloalkyl-Isocyanurate, welche cyclische Trimere der Diisocyanate darstellen, oder um Gemische mit ihren höheren, mehr als einen Isocyanuratring aufweisenden Homologen. Die Isocyanato-Isocyanurate haben im allgemeinen einen NCO-Gehalt von 10 bis 30 Gew.-%, insbesondere 15 bis 25 Gew.-%, und eine mittlere NCO-Funktionalität von 2,6 bis 4,5.
   Vor allem kommen als Verbindungen II Isocyanurate der allgemeinen Formel (IIa) 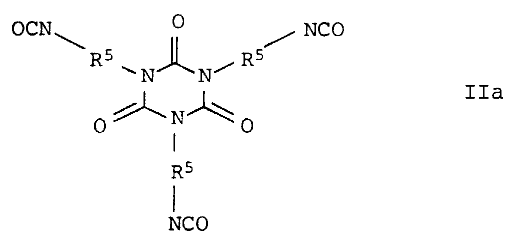 oder die sich davon ableitenden oligomeren Formen in Betracht, bei denen R⁵ die gleiche Bedeutung wie bei Verbindungen der Formel I hat.
(b) Uretdiongruppen enthaltende Diisocyanate mit aromatisch, aliphatisch und/oder cycloaliphatisch gebundenen Isocyanat-gruppen, vorzugsweise von Hexamethylendiisocyanat oder Isophorondiisocyanat abgeleitet. Bei Poly-Uretdiondiisocyanaten handelt es sich um Dimerisierungsprodukte von Diisocyanaten.
(c) Biuretgruppen aufweisende Polyisocyanate mit aliphatisch gebundenen Isocyanatgruppen, insbesondere Tris(6-isocyanatohexyl)biuret oder dessen Gemische mit seinen höheren Homologen. Diese Biuretgruppen aufweisenden Polyisocyanate weisen im allgemeinen einen NCO-Gehalt von 10 bis 30 Gew.-%, insbesondere von 18 bis 25 Gew.-% und eine mittlere NCO-Funktionalität von 2,8 bis 4,5 auf.
(d) Urethan- und/oder Allophanatgruppen aufweisende Polyisocyanate mit aliphatisch oder cycloaliphatisch gebundenen Isocyanatgruppen, wie sie beispielsweise durch Umsetzung von überschüssigen Mengen an Hexamethylendiisocyanat oder an Isophorondiisocyanat mit mehrwertigen Alkoholen wie Trimethylolpropan, Glycerin, 1,2-Dihydroxypropan oder deren Gemischen erhalten werden können. Diese Urethan- und/oder Allophanatgruppen aufweisenden Polyisocyanate haben im allgemeinen einen NCO-Gehalt von 12 bis 25 Gew.-% und eine mittlere NCO-Funktionalität von 2,5 bis 4,5.
(e) Oxadiazintriongruppen enthaltende Polyisocyante, vorzugsweise von Hexamethylendiisocyanat oder Isophorondiisocyanat abgeleitet. Solche Oxadiazintriongruppen enthaltenden Polyisocyanate sind aus Diisocyanat und Kohlendioxid herstellbar.
(f) Carbodiimid- oder Uretonimin-modifizierte Polyisocyanate.

Die Isocyanatgruppen der genannten Polyisocyanate (a) bis (f) können auch teilweise mit Monoalkoholen umgesetzt sein.

Verbindungen der Formel III, die besonders in Betracht kommen, sind beispielsweise aus der Deutschen Patentanmeldung mit dem Aktenzeichen P. 195 24 046.4 bekannt.

Was die Einheiten R¹, R², R⁵ und R⁶ betrifft, so sind bei den Verbindungen der Formel III die gleichen Einheiten bevorzugt, wie bei den entsprechenden Einheiten der Formel I. Die Einheiten R¹⁰, R¹¹, U und V in Formel III unterscheiden sich von den entsprechenden Einheiten R³, R⁴, X und Y in Formel I im allgemeinen lediglich darin, daß sie statt der Gruppe R^{Ia} die Gruppe R^{IIIa}, statt der Gruppe R^{Ib} die Gruppe R^{IIIb}, statt der Gruppe R^{Ic} die Gruppe R^{IIIc} und statt der Gruppe R^{Id} die Gruppe R^{IIId} tragen können.

Die Herstellung der Verbindungen der Formel I gelingt besonders einfach durch Umsetzung von Verbindungen der Formel IV 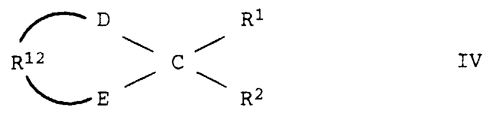 in der R¹, R², R¹², D und E die folgende Bedeutung haben:
- R¹, R²: wie in Formel I
- D, E: -O-, S, 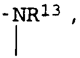 wobei R¹³ Wasserstoff, eine C₁- bis C₁₀-Alkylgruppe, die gegebenenfalls durch Sauerstoffatome in Etherfunktion unterbrochen ist, eine C₆- bis C₁₀-Arylgruppe oder eine C₇- bis C₁₀-Aralkylgruppe bedeutet,
- R¹²: eine C₁- bis C₁₀-Alkandiylgruppe, die zusammen mit D-CR¹R²-E einen 4 bis 7 gliedrigen Ring bildet,
wobei entweder bei der Einheit R¹² eines der Wasserstoffatome oder bei der Einheit 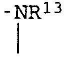 der Rest R¹³ durch einen
Rest R^{IVa}

-R⁶-OH

einen Rest R^{IVb}

-R⁶-NH₂

oder einen Rest R^{IVc} 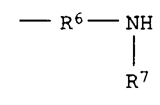 oder einen Rest R^{IVd}

-R⁶-SH

substituiert ist,
mit einer Verbindung der Formel V

OCN-R⁵-NCO V

bei einer Temperatur von 20 bis 140°C, wobei das Molverhältnis der Verbindung der Formel IV zur Verbindung der Formel V 1:1,5 bis 1:20 beträgt.

Die Umsetzung wird im allgemeinen in Substanz oder in Lösung bevorzugt bei Normaldruck vorgenommen.

Durch Wahl der entsprechenden Ausgangsverbindung IV mit den Substituenten der Formel R^{IVa}, R^{IVb}, R^{IVc} bzw. R^{IVd} können die erfindungsgemäßen Verbindungen der Formel I sowie Verbindungen der Formel III gezielt hergestellt werden. Die Einheiten D, E, R¹² in den Ausgangsverbindungen (Verbindungen IV) unterscheiden sich von den korrespondierenden Einheiten U, V, R¹⁰ (Verbindungen III) bzw. X, Y, R³ (Verbindungen I) in den Zielverbindungen im allgemeinen lediglich dadurch, daß sie verschiedene Substituenten R^{IVa}, R^{IVb}, R^{IVc}, R^{IVd} (Verbindungen IV), R^{IIIa}, R^{IIIb}, R^{IIIc,} R^{IIId} (Verbindungen III) bzw. R^{Ia}, R^{Ib}, R^{Ic}, R^{Id} (Verbindungen I) tragen. Die Kleinbuchstaben a, b, c oder d sind in den Bezeichnungen für die Ausgangs- und Zielverbindungen bzw. in den sie kennzeichnenden Resten so zugeordnet, daß sie bei den Ausgangs- und den daraus herstellbaren Zielverbindungen gleich sind (d.h. eine Verbindung mit dem Substituenten R^{IVa} führt beispielsweise zu Ia, wobei R¹, R², R⁵ und R⁷ sowohl in der Ausgangs- und in der Zielverbindung dieselbe Bedeutung haben). Wegen ihrer einfachen Herstellbarkeit sind Mischungen von Verbindungen I, II und gegebenenfalls III, die bei der Umsetzung von den entsprechenden Ausgangsverbindungen IV und V gebildet werden, besonders bevorzugt.

Die Umsetzung wird üblicherweise beendet, wenn die Ausgangsverbindung der Formel IV in der Reaktionsmischung praktisch quantitativ abreagiert ist.

Sonstige Reaktionsparameter sind dem Fachmann allgemein bekannt und können beispielsweise so gewählt werden, wie sie in der EP-A-0585835, 0496208, 069866, in den US-Patenten 5 124 427, 5 258 482, 5 290 902 sowie DE-A-4015155 für die Herstellung anderer Biurete, Allophanate und Isocyanurate beschrieben sind.

Üblicherweise wird die Umsetzung der Verbindungen IV und V in Gegenwart eines Katalysators, bevorzugt in Mengen von 10 bis 5000 Gew.-ppm, bezogen auf die Menge der eingesetzten Verbindungen V, durchgeführt.

Als Katalysatoren kommen die für die Trimerisierungsreaktion von Isocyanatgruppen allgemein bekannten Katalysatoren in Betracht, also beispielsweise die in der EP-A-0649866 beschriebenen quarternären Ammoniumhydroxide, z.B. N,N,N-Trimethyl-N-(2-hydroxipropyl)ammoniumhydroxid, oder die aus der EP-A-0182203 bekannten quarternären Ammoniumcarboxylate, z.B. N,N,N-Trimethyl-N-(2-hydroxypropyl)ammonium-2-ethylhexanoat, oder als Allophanatbildungskatalysatoren bekannte Zink-organische Verbindungen, z.B. Zink-Acetylacetonat oder Zink-2-Ethylcaproat.

Der Reaktionsfortschritt wird zweckmäßigerweise per Gelpermeationschromatographie (GPC) oder durch Bestimmung des NCO-Gehaltes der Reaktionsmischung verfolgt.

Die Umsetzung wird üblicherweise beendet, wenn die Ausgangsverbindung IV praktisch vollständig umgesetzt, das heißt per GPC nicht mehr detektierbar ist.

Die Beendigung der Umsetzung erfolgt üblicherweise durch Zusatz von Desaktivatoren. Als Desaktivatoren eignen sich beispielsweise anorganische oder organische Säuren, die entsprechenden Säurehalogenide und Alkylierungsmittel. Beispielhaft genannt seien Phosphorsäure, Monochloressigsäure, Dodecylbenzolsulfonsäure, Benzoylchlorid, Dimethylsulfat und vorzugsweise Dibutylphosphat sowie Di-2-ethylhexylphosphat. Die Desaktivierungsmittel können in Mengen von 1 bis 200 Mol-%, vorzugsweise 20 bis 100 Mol-%, bezogen auf die Mole an Katalysator, eingesetzt werden.

Nach Beendigung der Umsetzung trennt man zweckmäßigerweise den gegebenenfalls vorhandenen nicht-abreagierten Anteil der Verbindung der Formel V aus der Reaktionsmischung bevorzugt bis zu einem Gehalt von weniger als 1,0, besonders bevorzugt weniger als 0,5 Gew.-% ab. Falls die Verbindungen der Formel I von denen der Formel III abgetrennt werden sollen, so kann dies mittels Gelpermationschromatographie erfolgen. Eine Abtrennung erübrigt sich jedoch in den meisten Fällen, da beide Verbindungen in Zweikomponenten-Polyurethanbeschichtungsmitteln zu ähnlichen Zwecken eingesetzt werden können. Das resultierende Produkt ist in seiner Handhabung unproblematisch und kann im allgemeinen ohne spezielle Sicherheitsvorkehrungen verarbeitet werden.

Die erfindungsgemäßen Verbindungen der Formel I werden gegebenenfalls in Form ihrer Mischungen mit den Verbindungen II und III als B-Komponente in Zweikomponenten-Polyurethanbeschichtungsmitteln eingesetzt, die als A-Komponente im allgemeinen ein hydroxyfunktionelles Polymer (A) enthalten, das keine Isocyanatgruppen trägt.

Bei den hydroxyfunktionellen Polymeren (A) handelt es sich z.B. um Polymere mit einem Gehalt an Hydroxylgruppen von 0,1 bis 20, vorzugsweise 0,5 bis 10 Gew.-%. Das zahlenmittlere Molekulargewicht Mₙ der Polymeren beträgt vorzugsweise 1000 bis 100 000, besonders bevorzugt 2000 bis 10 000. Bei den Polymeren handelt es sich bevorzugt um solche, welche zu mehr als 50 Gew.-% aus C₁-C₂₀-Alkyl(meth)acrylat, Vinylaromaten mit bis zu 20 C-Atomen, Vinylestern von bis zu 20 C-Atomen enthaltenden Carbonsäuren, Vinylhalogeniden, nicht aromatischen Kohlenwasserstoffen mit 4 bis 8 C-Atomen und 1 oder 2 Doppelbindungen, ungesättigten Nitrilen und deren Mischungen bestehen. Besonders bevorzugt sind die Polymeren, die zu mehr als 60 Gew.-% aus C₁-C₁₀-Alkyl(meth)acrylaten, Styrol oder deren Mischungen bestehen.

Darüber hinaus enthalten die Polymeren (A) hydroxyfunktionelle Monomere entsprechend dem obigen Hydroxylgruppengehalt und gegebenenfalls weitere Monomere, z.B. ethylenisch ungesättigte Säuren, insbesondere Carbonsäuren, Säureanhydride oder Säureamide.

Weitere Polymere (A) sind z.B. Polyesterole, wie sie durch Kondensation von Polycarbonsäuren, insbesondere Dicarbonsäuren mit Polyolen, insbesondere Diolen erhältlich sind.

Weiterhin sind als Polymere (A) auch Polyetherole geeignet, die durch Addition von Ethylenoxid, Propylenoxid oder Butylenoxid an H-aktive Komponenten hergestellt werden. Ebenso sind Polykondensate aus Butandiol geeignet.

Bei den Polymeren (A) kann es sich natürlich auch um Verbindungen mit primären der sekundären Aminogruppen handeln.

Genannt seien z.B. sogenannte Jeffamine, d.h. mit Aminogruppen terminierte Polyetherole oder Oxazolidine.

Neben den vorstehend aufgeführten A- und B-Komponenten können in den Beschichtungsmitteln weiterhin sonstige Polyisocyanate und Verbindungen mit gegenüber Polyisocyanaten reaktionsfähigen Gruppen enthalten sein, wie sie üblicherweise in Zweikomponenten-Beschichtungsmassen vorhanden sind. Beispielsweise kommen hierfür insbesondere die Isocyanate in Betracht, die zur Herstellung der Verbindungen der Formel I eingesetzt werden.

B-Komponenten für Zweikomponenten-Polyurethanbeschichtungsmassen enthalten im allgemeinen
- 0,2 bis 99,9 mol-% Isocyanat-Gruppen in Form der Verbindung der Formel I
- 0,1 bis 99,8 mol-% Isocyanat-Gruppen in Form einer Verbindung der Formel II und
- 0 bis 58,2 mol-% Isocyanat-Gruppen in Form einer Verbindung der Formel III.

Bevorzugte Zweikomponenten-Polyurethanbeschichtungsmassen enthalten als A-Komponente
ein Polymer (A) mit mindestens 2 gegenüber Isocyanatgruppen reaktiven Gruppen, bevorzugt alkoholischen Hydroxylgruppen,
wobei das molare Verhältnis der Summe gebildet aus den Einheiten X, Y, U, V in den Verbindungen der Formel I und III sowie den gegenüber Isocyanatgruppen reaktiven Gruppen des Polymeren (A) zu der Summe der Isocyanatgruppen in den Verbindungen der Formel I, II und III 0,6:1 bis 1,4:1, bevorzugt 0,7:1 bis 1,3:1 beträgt.

Komponenten B, die sich für den Einsatz in Zweikomponenten-Polyurethanbeschichtungsmitteln eignen, erhält man direkt, wenn man die Verbindungen IV mit den Verbindungen V im Molverhältnis 1:50, bis 1:1,5 umsetzt, bis die gegenüber Isocyanaten reaktiven Gruppen R^{IV} praktisch vollständig abreagiert sind, und anschließend den nicht-umgesetzten Anteil der Verbindungen V aus dem Reaktionsgemisch entfernt.

Mischungen, die als Einkomponenten-Polyurethanbeschichtungsmassen eingesetzt werden können, enthalten üblicherweise
- 0,2 bis 100, bevorzugt 0,2 bis 80 mol-% Isocyanatgruppen in Form der Verbindung der Formel I
- 0 bis 58,2, bevorzugt 10 bis 49,9 mol-% Isocyanat-Gruppen in Form einer Verbindung der Formel II und
- 0 bis 58,2, bevorzugt 10 bis 49,9 mol-% Isocyanat-Gruppen in Form einer Verbindung der Formel III.

Mit Vorteil werden solche Beschichtungsmassen eingesetzt, die
- pro Mol einer Verbindung der Formel I
- 0,1 bis 10, bevorzugt 0,2 bis 5 mol einer Verbindung der Formel II und
- pro Mol NCO-Gruppen in der Verbindung der Formel II 0,6 bis 1,4, bevorzugt 0,9 bis 1,1 mol einer Verbindung der Formel III
enthalten.

Bevorzugt sind solche Mischungen, deren realer Gehalt an Isocyanat-Gruppen (NCO-Gruppen) 1 bis 40, besonders bevorzugt 5 bis 25 Gew.-% beträgt (dabei wurde mit einem Molekulargewicht der NCO-Gruppen von 42 gerechnet) und einem fiktiven NCO-Gehalt von -6 bis +6, besonders bevorzugt -3 bis +3 Gew.-%.

Den realen NCO-Gehalt in Prozent erhält man durch Messung der molaren Menge der NCO-Gruppen pro Gewichtseinheit, der z.B. durch allgemein bekannte Titrationsmethoden ermittelt werden kann, und Multiplikation dieses Wertes mit 100 und 42 (Molekulargewicht von -NCO). Eine übliche Titrationsmethode ist in der DIN 53185 beschrieben.

Der fiktive NCO-Gehalt ergibt sich rechnerisch, indem man von der gemessenen molaren Menge der NCO-Gruppen pro Gewichtseinheit die molare Menge an NCO-Gruppen pro Gewichtseinheit abzieht, die der molaren Menge an Einheiten X, Y, U und V pro Gewichtseinheit entspricht und diesen Wert mit 4200 multipliziert.

Der fiktive NCO-Gehalt in Prozent ist also der NCO-Gehalt, der sich ergäbe, wenn die geschützten gegenüber NCO-reaktiven Gruppen (Einheiten X, Y, U und V) quantitativ mit den NCO-Gruppen reagiert hätten und ist bei den Verbindungen I gleich 0.

Derartige Einkomponenten-Systeme sind praktisch unbegrenzt lagerbar und vernetzen erst bei Zutritt von Wasser, beispielsweise in Form von Luftfeuchtigkeit, da durch das Wasser die geschützten Einheiten X, Y, U und V entschützt, d.h. in gegenüber Isocyanat-gruppen reaktive Gruppen überführt werden. Durch die Wahl des definitionsgemäßen stöchiometrischen Verhältnisses der Einheiten X, Y, U und V zu Isocyanatgruppen wird gewährleistet, daß die Verbindungen ein hochmolekulares Netzwerk ausbilden, was Voraussetzung für das hohe anwendungstechnische Niveau der Beschichtungen ist.

Die Polyisocyanate II können auch ganz oder teilweise durch andere mit der oder den Reaktivkomponenten reaktionsfähigen Verbindungen ersetzt werden. In Betracht kommen z.B. Polyepoxide, Verbindungen mit Säureanhydridgruppen oder N-Methylolgruppen bzw. veretherte N-Methylolgruppen enthaltende Verbindungen, z.B. Harnstoff- oder Melaminharze, welche mit den deblockierten Gruppen X, Y, U und V der Verbindung I und III reagieren können.

Die erfindungsgemäßen Beschichtungsmassen können weiterhin noch organische Lösemittel, z.B. Xylol, Butylacetat, Methylisobutylketon, Methoxypropylacetat, N-Methylpyrrolidon enthalten. Mit Lösemittel wird die zur Verarbeitung, d.h. zum Auftragen auf Substrate gewünschte niedrige Viskosität der Beschichtungsmasse eingestellt. Durch die Verbindungen I wird dazu deutlich weniger Lösemittel benötigt, d.h. die gewünschte niedrigere Viskosität wird bei höheren Festgehalten erreicht.

Die Beschichtungsmassen können natürlich weitere, in der Beschichtungstechnologie übliche Zusatzstoffe, z.B. Pigmente, Füllstoffe, Verlaufshilfsmittel etc. enthalten.

Sie können weiterhin Katalysatoren für die Urethanbildung, z.B. Dibutylzinndilaurat, enthalten.

Die Herstellung der Zweikomponenten-Polyurethanbeschichtungsmittel kann in bekannter Weise erfolgen. Gewöhnlich werden die A- und die B-Komponente vor dem Auftrag der Beschichtungsmittel auf ein Substrat gemischt. Mit Lösungsmittel kann die gewünschte Viskosität eingestellt werden.

Die Herstellung der Einkomponenten-Polyurethanbeschichtungsmittel kann zu einem beliebigen Zeitpunkt vor deren Applikation erfolgen, da die Vernetzung nicht spontan erfolgen kann, denn die gegenüber den Isocyanatgruppen reaktiven Amin-, Thiol- bzw. Hydroxylgruppen liegen in geschützter Form, d.h. als Gruppen X, Y und gegebenenfalls U und V vor.

Die Vernetzung erfolgt nach der Applikation, wenn die Beschichtungsmittel mit Wasser oder Luftfeuchtigkeit in Kontakt kommen.

Unter Einfluß von Wasser werden die blockierten mit Isocyanat reaktiven Gruppen der Verbindung I und III freigesetzt. Danach verläuft die Reaktion der deblockierten Gruppen der Formel I und III mit den Polyisocyanaten bei Raumtemperatur oder bei erhöhter Temperatur in bekannter Weise.

Die Ein- und Zweikomponenten-Beschichtungsmittel können in üblicher Weise durch Spritzen, Gießen, Walzen, Streichen, Rakeln etc. auf Substrate aufgebracht werden.

Die Beschichtungsmittel eignen sich insbesondere für Werkstücke mit Oberflächen aus Metall, Kunstoff, Holz, Holzwerkstoffen etc.

Die erhaltenen Beschichtungen haben sehr gute mechanische Eigenschaften, insbesondere eine hohe Härte, Flexibilität und Chemikalienbeständigkeit.

Vor allem weisen die erfindungsgemäßen Verbindungen der Formel I den Vorteil auf, daß sich mit ihnen hochwertige Ein- und Zweikomponenten-Polyurethanbeschichtungsmittel herstellen lassen, die eine besonders niedrige Viskosität aufweisen.

Die Mischungen aus Verbindungen I und die Mischungen aus den Verbindungen I, II und III eignen sich auch als Reaktivverdünner in Zweikomponenten-Polyurethan-Systemen, da sie an der Vernetzungsreaktion teilzunehmen vermögen, aber die stöchiometrischen Verhältnisse der NCO-Gruppen zu den mit NCO-Gruppen reaktionsfähigen Gruppen praktisch kaum verändern.

### Beispiele

### Ausgangsverbindungen der Formel IV

IV.1 2,2-Dimethyl-4-hydroxymethyl-1,3-dioxolan (Isopropylidenglycerin) 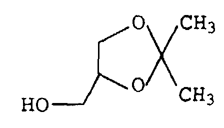
IV.2 4-Aminomethyl-2,2-dimethyl-1,3-dioxolan 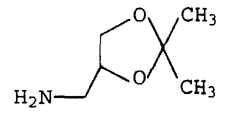 bekannt aus F.S. Gibson, M.S. Park und H. Rapoport, J. Org. Chem. 1994,59,7503-7507
IV.3 Reaktionsprodukt aus 3 Mol Ethylenoxid und Verbindung IV.1
   Zur Herstellung von IV.3 wurden in einem 51-Reaktor, geeignet zur Herstellung von Polyetherolen, 1060 g (8 mol) Isopropylidenglycerin (IV.1) vorgelegt, 4 g Kalium-tert.-butylat zugefügt und auf 110°C erhitzt. Bei dieser Temperatur wurden 24 mol Ethylenoxid zugegeben. Die Reaktion wurde weitergeführt bis zur Druckkonstanz.
   Anschließend wurde für 30 min. Vakuum angelegt. Nach der Entmonomerisierung wurde mit Stickstoff belüftet, auf 50°C abgekühlt und das Produkt abgelassen. Die Aufarbeitung zur Entfernung des Alkali erfolgte durch Zugabe von 3 Gew.% eines Mg-Silikats (Ambusol, Kationenaustauscher) und Erhitzen für 2h auf 100°C. Das Silikat wurde abfiltriert und das Endprodukt mit 0.15 Gew.% 2,6-Di-tert.butyl-p-kresol (Kerobit TBK) stabilisiert.
   OH-Zahl = 216
IV.4 2,2-Dimethyl-5-ethyl-5-hydroxy-methyl-1,3-dioxan (Isopropyliden-TMP) 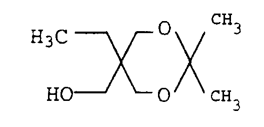
   Zur Herstellung von IV.4 wurden 250 g Trimethylolpropan zusammen mit 750 ml Petrolether (Siedebereich 30-75°C), 750 ml Aceton und 0.15 g p-Toluolsulfonsäure-Monohydrat 24 h am Rückfluß erhitzt. Danach wurde über einen Wasserabscheider das entstandene Reaktionswasser entfernt. Die Lösung wurde abgekühlt, 0.5 g Natriummethanolat zugegeben und 1 h bei Raumtemperatur gerührt. Die Lösung wurde filtriert, das Lösungsmittel am Rotationsverdampfer entfernt und der Rückstand im Vakuum destilliert. Ausb. 78 % d. Theor., Siedep. 71-72°C (0.5 mbar)
IV.5 N-(2-Hydroxyethyl)-2-isopropyl-oxazolidin 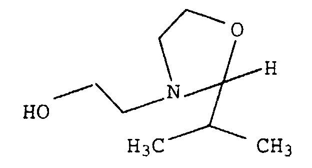
   Bekannt aus DE-A-2 245 636

### A. B-Komponenten für Zweikomponenten Beschichtungsmittel enthaltend Verbindungen I

2000 g Hexamethylendiisocyanat (HDI) wurden unter Stickstoffbedeckung vorgelegt und die entsprechende Menge der Verbindung IV.1 bis IV.5 laut Tabelle 1 zugesetzt. Man erwärmte die Mischung auf 80°C, gab 0.4 g des Katalysators DABCO TMR 1 (Handelsname der Fa. Air Products, N-(2-Hydroxypropyl)-trimethylammonium-2-ethylhexanoat) zu, ließ bei dieser Temperatur reagieren und stoppte die Reaktion bei einem NCO-Gehalt der Mischung von 39 - 41 Gew.% durch Zugabe von 0.4 g Di-2-ethylhexylphosphat. Bei Einsatz des Produktes IV.2 wurde ohne Katalyse auf 120°C erhitzt. Das Reaktionsgemisch wurde anschließend zur Entfernung von monomerem HDI im Dünnschichtverdampfer bei 165°C Öltemperatur und 2.5 mbar destilliert. Der HDI-Restmonomergehalt des Endproduktes lag danach unter 0.3 Gew.%.

### Vergleichsprodukte, Stand der Technik

- Produkt 1:: BASONAT® HI 100, realer NCO-Gehalt = 22.0 %, Visk. 25°C = 3900 mPas (Polyisocyanat, BASF AG)
- Produkt 2:: BASONAT®P LR 8901, realer NCO-Gehalt = 20.0 %, Visk. 25°C = 790 mPas (niederviskoses Polyisocyanat, BASF AG)

Erfindungsgemäße Produkte

**Tabelle 1:**

| B-Komponenten | | | | |
|---|---|---|---|---|
| Produkt | Ausgangsverbindung IV | Molverhältnis* | NCO-Gehalt** (Gew.-%) | Visk. 25°C (mPas) |
| 3 | IV.1 | 2,5 | 21,1 | 1590 |
| 4 | IV.1 | 5,0 | 20,1 | 810 |
| 5 | IV.1 | 10,0 | 18,4 | 680 |
| 6 | IV.2 | 10,0 | 18,5 | 1310 |
| 7 | IV.3 | 10,0 | 15,9 | 460 |
| 8 | IV.4 | 2,5 | 20,9 | 2150 |
| 9 | IV.4 | 5,0 | 19,6 | 2040 |
| 10 | IV.4 | 10,0 | 17,7 | 2260 |
| 11 | IV.5 | 2,5 | 20,5 | 1540 |
| 12 | IV.5 | 5,0 | 19,1 | 1040 |
| 13 | IV.5 | 10,0 | 17,0 | 1410 |

| | | | | |
|---|---|---|---|---|
| * Anteil der Ausgangsverbindung der Formel IV bezogen auf das HDI [mol-%] | | | | |
| ** realer NCO-Gehalt | | | | |

### Herstellung und Prüfung von Zweikomponenten-Klarlacken aus den erfindungsmäßen Polyisocyanaten

Die erfindungsgemäßen Produkte aus Tabelle 1 wurden mit einem hydroxifunktionellen Vinylpolymerisat (LUMITOL® H 136, Festgehalt = 70 %, OHZ = 136, BASF AG) entsprechend den stöchiometrischen NCO/OH-Verhältnissen gemischt und zur Beschleunigung der Aushärtung mit 0,1 %(bezogen auf den Festkörperanteil) Dibutylzinndilaurat (DBTL, Merck) katalysiert. Die Einstellung auf eine Applikationsviskosität von 20 s (DIN 53 211 Becher 4 mm Auslaufdüse) erfolgte mit Butylacetat. Die Lackfestgehalte wurden nach DIN V 53 216 1. Teil bestimmt, die VOC-Werte aus Masse/Volumen-Verhältnissen berechnet.

Mit einem Filmziehrahmen wurden Beschichtungen mit einer Naßfilmdicke von 200 µm auf Glasplatten aufgetragen. Die so erhaltenen Klarlacke wurden 7 Tage unter Normklima gehärtet. Die erhaltenen Lackeigenschaften sind in Tabelle 2 zusammengefaßt. Als Vergleich wurden Klarlacke mit BASONAT® HI 100 und BASONAT® P LR 8901 (BASF AG) geprüft.

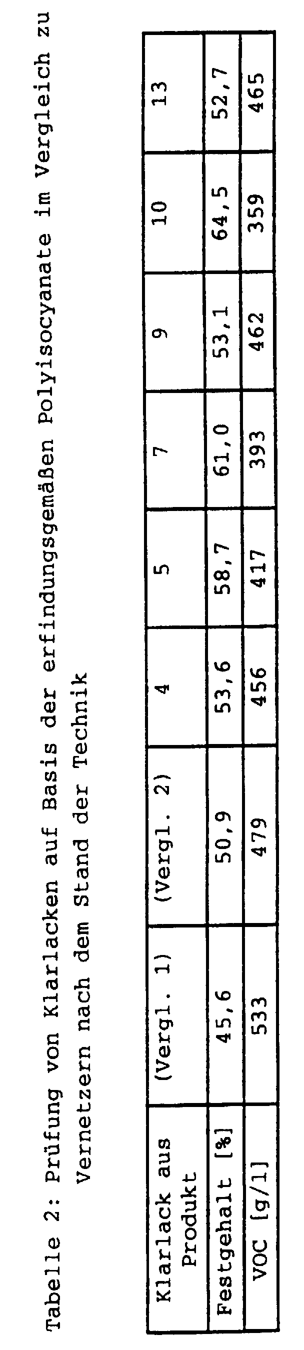

Die mit den erfindungsgemäßen Polyisocyanatvernetzern hergestellten Lacke zeigen neben hervorragender Härte (Kratzfestigkeit) und Flexibilität einen gegenüber dem Stand der Technik verbesserten Lackfestgehalt, bzw. niedrigeren Lösemittelanteil (VOC = volatile organic compounds).

### B. Einkomponenten-Polyurethanbeschichtungsmittel und Reaktivverdünner enthaltend Verbindungen I

### Herstellung der Produkte 14 bis 17

6 mol Hexamethylendiisocyanat (HDI) wurden unter Stickstoffbedeckung vorgelegt und 1,2 mol der Verbindung IV.1 bis IV.5 laut Tabelle 3 zugesetzt. Man erwärmte die Mischung auf 80°C, gab 150 Gew.-ppm des Katalysators DABCO TMR 1 (Handelsname der Fa. Air Products, N-(2-Hydroxypropyl)-trimethyl-ammonium-2-ethylhexanoat) zu, ließ bei dieser Temperatur reagieren und stoppte die Reaktion bei einen NCO-Gehalt der Mischung von 30 - 32 Gew.-% durch Zugabe von 160 Gew.-ppm Di-2-ethylhexylphosphat bezogen auf HDI. Das Reaktionsgemisch wurde anschließend zur Entfernung von monomerem HDI im Dünnschichtverdampfer bei 165°C Öltemperatur und 2.5 mbar destilliert. Der HDI-Restmonomergehalt des Endproduktes lag danach unter 0.3 Gew.-%.

### Herstellung der Produkte 18 bis 20

6 mol IPDI wurden unter Stickstoffbedeckung vorgelegt und 1.2 mol der Komponente IV zugesetzt. Man erwärmte die Mischung auf 70°C, gab 1200 Gew.-ppm (bezogen auf Diisocyanat) des Katalysators DABCO TMR 1 zu, ließ bei dieser Temperatur reagieren und stoppte die Reaktion bei einem NCO-Gehalt der Mischung von 25.5 - 26.5 Gew.-% durch Zugabe von 1300 Gew.-ppm (bezogen auf Diisocyanat) Di-2-ethylhexylphosphat. Das Reaktionsgemisch wurde anschließend zur Entfernung vom monomeren IPDI im Dünnschichtverdampfer bei 165°C Öltemperatur und 2.5 mbar destilliert.

### Herstellung der Produkte 21 und 22

6 mol des Diisocyanates wurden unter Stickstoffbedeckung vorgelegt und 1.2 mol Isopropylidenglycerin (IV.1) zugesetzt. Man erwärmte die Mischung auf 80°C, gab 250 Gew.-ppm (bezogen auf Diisocyanat) des Katalysators DABCO TMR 1 zu, ließ bei dieser Temperatur reagieren und stoppte die Reaktion bei einem NCO-Gehalt der Mischung von 24 Gew.-% bei BEPDI bzw. 26 Gew.-% bei IPCI durch Zugabe von 260 Gew.-ppm (bezogen auf Diisocyanat) Di-2-ethylhexylphosphat. Das Reaktionsgemisch wurde anschließend zur Entfernung vom monomeren Diisocyanat im Dünnschichtverdampfer bei 165°C Öltemperatur und 2.5 mbar destilliert.

**Tabelle 3**

| Produkt Nr. | Isocyanat | Ausgangsverbindung IV | NCO fiktiv [Gew.-%] | NCO real [Gew.-%] | Visk. [mPas] |
|---|---|---|---|---|---|
| 14 | HDI | IV.1 | -1,7 | 16,2 | 490 (25°C) |
| 15 | HDI | IV.3 | -0,3 | 13,8 | 510 (25°C) |
| 16 | HDI | IV.4 | -1,3 | 15,2 | 2310 (25°C) |
| 17 | HDI | IV.5 | -1,6 | 15,4 | 1220 (25°C) |
| 18 | IPDI | IV.1 | -1,2 | 13,4 | 1470* (50°C) |
| 19 | IPDI | IV.4 | +0,1 | 13,7 | 2950* (50°C) |
| 20 | IPDI | IV.5 | -1,8 | 12,1 | 3040* (50°C) |
| 21 | IPCI | IV.1 | 0,0 | 15,4 | 26240 (25°C) |
| 22 | BEPDI | IV.1 | -1,8 | 12,0 | 19300 (25°C) |

| | | | | | |
|---|---|---|---|---|---|
| * = 90%ig in Butylacetat HDI = Hexamethylendiisocyanat IPDI = Isophorondiisocyanat IPCI = 2-Isocyanatopropylcyclohexylisocyanat BEPDI = 2-Butyl-2-ethylpentamethylendiisocyanat | | | | | |

### Herstellung und Prüfung von Einkomponenten-Beschichtungsmitteln

Die erfindungsgemäßen Produkte aus Tabelle 3 wurden (bei negativen fiktiven NCO-Gehalten entsprechend der Stöchiometrie mit Basonat® HI 100 gemischt und) zur Beschleunigung der Aushärtung mit 0,1 % Dibutylzinndilaurat (DBTL, Merck) versetzt. Die Einstellung auf eine Applikationsviskosität von 20 s (DIN 53 211 Becher 4 mm Auslaufdüse) erfolgte mit Butylacetat. Die Lackfestgehalte wurden nach DIN V 53 216 1. Teil bestimmt, die VOC-Werte aus Masse/Volumen-Verhältnissen berechnet.

Die Lackfestgehalte sind in Tabelle 4 zusammengefaßt. Als Vergleich wurde ein Klarlack auf Basis Lumitol® H 136 (Hydroxiacrylatharz, 70%ig in Butylacetat, OHZ = 135, BASF AG), mit BASONAT® HI 100 (Polyisocyanat, 100%ig, realer NCO-Gehalt = 22%, BASF AG) vernetzt, geprüft.

**Tabelle 4**

| Klarlack aus Isocyanat-Nr. | Vergleich | 14 | 15 | 16 | 17 | 18 | 20 | 21 | 22 |
|---|---|---|---|---|---|---|---|---|---|
| Festgehalt [%] | 45,6 | 81,2 | 80,0 | 77,4 | 77,0 | 69,9 | 69,3 | 76,6 | 75,3 |
| VOC [g/l] | 533 | 201 | 213 | 238 | 240 | 308 | 309 | 249 | 251 |

Die mit den erfindungsgemäßen Isocyanatvernetzern hergestellten Lacke zeigen neben hervorragender Härte (Kratzfestigkeit) einen gegenüber dem Stand der Technik deutlich verbesserten Lackfestgehalt, bzw. niedrigeren Lösemittelanteil (VOC = volatile organic compounds).

### Herstellung und Prüfung von Mischungen als Reaktivverdünner

Isocyanat Nr. 14 wurde mit dem Lack des Vergleichsbeispiels aus Tabelle 4 in verschiedenen Verhältnissen gemischt und die Mischung zur Beschleunigung der Aushärtung mit 0,1 % Dibutylzinndilaurat (DBTL, Merck) katalysiert. Die Einstellung auf eine Applikationsviskosität von 20 s (DIN 53 211 Becher 4 mm Auslaufdüse) erfolgte mit Butylacetat. Die Lackfestgehalte wurden nach DIN V 53 216 1. Teil bestimmt, die VOC-Werte aus Masse/Volumen-Verhältnissen berechnet. Mit einem Filmziehrahmen wurden Beschichtungen mit einer Naßfilmdicke von 150 µm auf Glasplatten aufgetragen. Die so erhaltenen Klarlacke wurden 7 Tage unter Normklima gehärtet. Die resultierenden Lackeigenschaften sind in Tabelle 5 zusammengefaßt.

**Tabelle 5**

| | | | | | |
|---|---|---|---|---|---|
| Mischungsverhältnis* Isocyanat-Nr. 14 | 0 | 50 | 70 | 85 | 100 |
| Standardlack | 100 | 50 | 30 | 15 | 0 |
| | | | | | |
| Mischungsverhältnis** Polyisocyanat-Nr. 1 | 0 | 56,4 | 75,1 | 88,0 | 100 |
| Standardlack | 100 | 43,6 | 24,9 | 12,0 | 0 |
| | | | | | |
| Erichsentiefung [mm] | 8,9 | 9,9 | 10 | 10 | 10 |
| Haftung/Gitterschnitt | 0,5 | 0 | 0 | 0 | 0 |
| Kratzfestigkeit | 0 | 0 | 0 | 0 | 0 |
| Festgehalt [%] | 45,6 | 62,4 | 69,5 | 75,5 | 81,2 |
| VOC [g/l] | 533 | 382 | 316 | 259 | 201 |

| | | | | | |
|---|---|---|---|---|---|
| * angegeben ist das Gewichtsverhältnis | | | | | |
| ** angegeben ist das Gewichtsverhältnis, bezogen auf den jeweiligen Festkörperanteil der Komponenten | | | | | |

## Patentansprüche

1. Verbindungen mit Isocyanatgruppen und verkappten gegenüber Isocyanatgruppen reaktiven Gruppen der Formel I 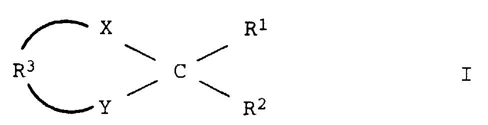 in denen R¹, R², R³, X und Y die folgende Bedeutung haben:
R¹, R² Wasserstoff, C₁- bis C₁₀-Alkyl, C₆- bis C₁₀-Aryl, C₇- bis C₁₀-Aralkyl, oder R¹ und R² bilden gemeinsam C₃- bis C₁₀-Alkandiyl,
X, Y -O-, -S-, 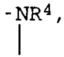 wobei R⁴ Wasserstoff, eine C₁- bis C₂₀-Alkylgruppe, die gegebenenfalls durch Sauerstoffatome in Etherfunktion unterbrochen ist, eine C₆- bis C₁₀-Arylgruppe oder eine C₇- bis C₁₀-Aralkylgruppe bedeutet,
R³ eine C₁- bis C₁₀-Alkandiylgruppe, die zusammen mit der Einheit -X-CR¹R²-Y- einen 4 bis 7gliedrigen Ring bildet,
wobei entweder bei der Einheit R³ eines der Wasserstoffatome oder bei der Einheit 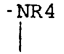 der Rest R⁴ durch eine
Allophanat-Gruppe R^{Ia} 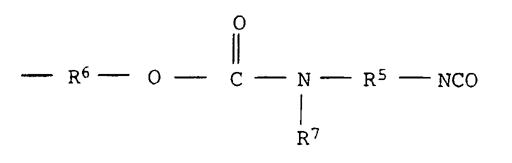 in der
R⁵ eine divalente aliphatische, alicyclische, araliphatische oder aromatische C₂- bis C₂₀-Kohlenwasserstoffeinheit und
R⁶ eine Einfachbindung oder eine divalente aliphatische, alicyclische, araliphatische oder aromatische C₁- bis C₂₀-Kohlenwasserstoffeinheit oder eine Mono- oder Poly-(C₂- bis C₄-Alkylenoxid)-Einheit und
R⁷ ein Carbamoylrest 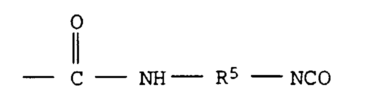 bedeutet
oder eine Biuret-Gruppe R^{Ib} 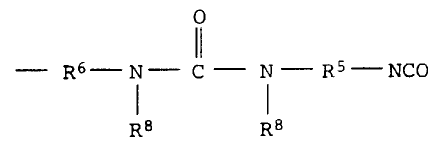 in der einer der Reste R⁸ Wasserstoff und der andere Rest die gleiche Bedeutung wie R⁷ hat
oder eine Biuret-Gruppe R^{Ic} 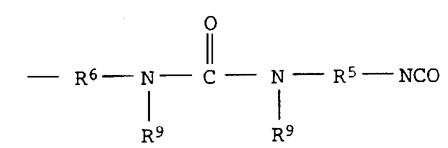 in der einer der Reste R⁹ die gleiche Bedeutung wie R⁷ und der andere die gleiche Bedeutung wie R¹ hat
oder eine Thioallophanat-Gruppe R^{Id} 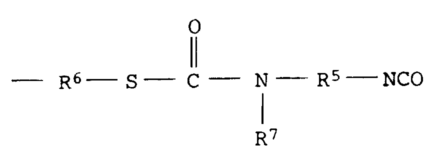 ersetzt ist.

2. Mischungen, enthaltend
a) 0,2 bis 100 mol-% Isocyanat-Gruppen in Form der Verbindung der allgemeinen Formel I,
b) 0 bis 99,8 mol-% Isocyanat-Gruppen in Form eines üblichen Isocyanates mit im Mittel wenigstens 2 Isocyanat-Gruppen (Verbindung II)
c) 0 bis 58,2 mol-% Isocyanat-Gruppen in Form einer Verbindung der Formel III 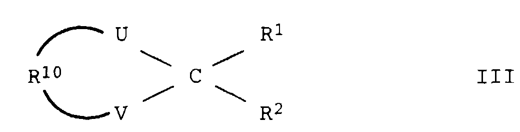 in der R¹, R², R¹⁰, U und V die folgende Bedeutung haben:
R¹, R² die bei Formel I angegebene Bedeutung
U, V -O-, -S-, 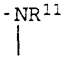 wobei R¹¹ Wasserstoff, eine C₁- bis C₁₀-Alkylgruppe, die gegebenenfalls durch Sauerstoffatome in Etherfunktion unterbrochen ist, eine C₆- bis C₁₀-Arylgruppe oder eine C₇- bis C₁₀-Aralkylgruppe,
R¹⁰ eine C₁- bis C₁₀-Alkandiylgruppe, die zusammen mit -U-CR¹R²-V- einen 4- bis 7-gliedrigen Ring bildet, wobei entweder bei der Einheit R¹⁰ eines der Wasserstoffatome oder bei der Einheit 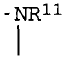 der Rest R¹¹
durch eine Urethan-Gruppe R^{IIIa} 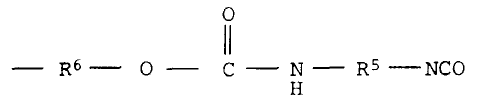 oder eine Harnstoff-Gruppe R^{IIIb} 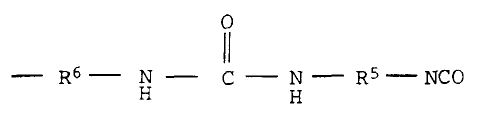 oder eine Harnstoff-Gruppe R^{IIIc} 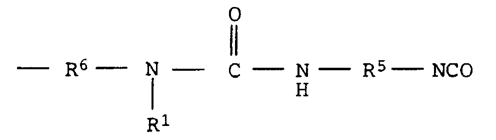 oder eine Thiourethan-Gruppe R^{IIId} 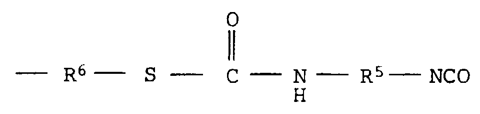 wobei R^{1,} R⁵ und R⁶ die in Formel I angegebene Bedeutung haben,
substituiert ist.

3. Mischungen nach Anspruch 2, wobei es sich bei den Verbindungen II um solche der allgemeinen Formel (IIa) 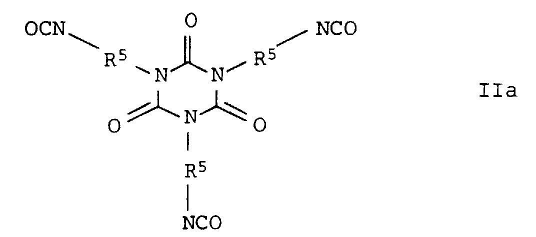 oder der sich daraus ableitenden oligomeren Formen handelt.

4. Verfahren zur Herstellung der Mischungen nach Anspruch 3, dadurch gekennzeichnet, daß man eine Verbindung der Formel IV, 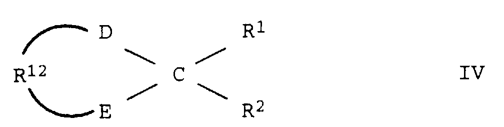 in der R¹, R², R¹², D und E die folgende Bedeutung haben:
R¹, R² wie in Formel I 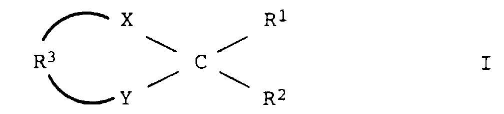
D, E -O-, -S-, 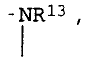 wobei R¹³ Wasserstoff, eine C₁- bis C₁₀-Alkylgruppe, die gegebenenfalls durch Sauerstoffatome in Etherfunktion unterbrochen ist, eine C₆- bis C₁₀-Arylgruppe oder eine C₇- bis C₁₀-Aralkylgruppe bedeutet,
R¹² eine C₁- bis C₁₃-Alkandiylgruppe, die zusammen mit -D-CR¹R²-E- einen 4- bis 7-gliedrigen Ring bildet,
wobei entweder bei der Einheit R¹² eines der Wasserstoffatome oder bei der Einheit 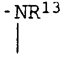 der Rest R¹³ durch einen
Rest R^{IVa},
-R⁶-OH
einen Rest R^{IVb},
-R⁶-NH₂
oder einen Rest R^{IVc} 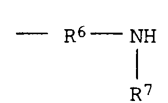 oder einen Rest R^{IVd}
-R⁶-SH
substituiert ist mit einer Verbindung der Formel V
OCN-R⁵-NCO
bei einer Temperatur von 20 bis 140°C umsetzt, wobei das Molverhältnis der Verbindung der Formel IV zur Verbindung der Formel V 1 : 1,5 bis 1 : 20 beträgt.

5. Verfahren nach Anspruch 4, dadurch gekennzeichnet, daß man nach Beendigung der Umsetzung die nicht-abreagierten Anteile der Verbindung der Formel V bis auf einen Gehalt von weniger als 1 Gew.-% abtrennt.

6. B-Komponente für Zweikomponenten-Polyurethanbeschichtungsmassen, enthaltend
- 0,2 bis 99,9 mol-% Isocyanat-Gruppen in Form der Verbindung der Formel I
- 0,1 bis 99,8 mol-% Isocyanat-Gruppen in Form einer Verbindung der Formel II und
- 0 bis 58,2 mol-% Isocyanat-Gruppen in Form einer Verbindung der Formel III.

7. Zweikomponenten-Polyurethanbeschichtungsmassen, enthaltend
- eine B-Komponente nach Anspruch 6 und
- eine A-Komponente mit mindestens 2 gegenüber NCO reaktiven Gruppen
mit der Maßgabe, daß das molare Verhältnis der Summe gebildet aus den Einheiten X, Y, U, V in den Verbindungen der Formel I und III sowie den mit NCO reaktiven Gruppen der A-Komponente zu der Summe der Isocyanatgruppen in den Verbindungen der Formel I, II und III 0,6:1 bis 1,4:1 beträgt.

8. Einkomponenten-Polyurethanbeschichtungsmassen, enthaltend
- 0,2 bis 100 mol-% Isocyanat-Gruppen in Form der Verbindung der Formel I
- 0 bis 58,2 mol-% Isocyanat-Gruppen in Form einer Verbindung der Formel II und
- 0 bis 58,2 mol-% Isocyanat-Gruppen in Form einer Verbindung der Formel III.

9. Beschichtungsverfahren, dadurch gekennzeichnet, daß man einen Gegenstand mit einer Zweikomponenten-Polyurethanbeschichtungsmasse nach Anspruch 6 oder 7 beschichtet.

10. Beschichtungsverfahren, dadurch gekennzeichnet, daß man einen Gegenstand mit einer Einkomponenten-Polyurethanbeschichtungsmasse nach Anspruch 8 beschichtet.

## Claims

1. An isocyanato compound with capped, isocyanate-reactive groups, of the formula I 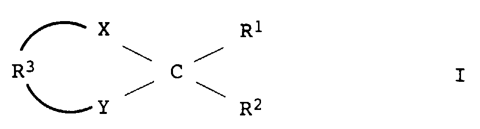 where
R¹ and R² are hydrogen, C₁-C₁₀-alkyl, C₆-C₁₀-aryl or C₇-C₁₀-aralkyl or together form C₃-C₁₀-alkanediyl,
X and Y are -O-, -S- or 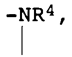 where R⁴ is hydrogen, C₁-C₂₀-alkyl which is uninterrupted or interrupted by oxygen atoms in ether function, or is C₆-C₁₀-aryl or C₇-C₁₀-aralkyl,
R³ is C₁-C₁₀-alkanediyl which together with -X-CR¹R²-Y- forms a 4-7-membered ring,
in which either one hydrogen in R³ or the radical R⁴ in 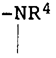 is replaced by
an allophanate group R^{Ia} 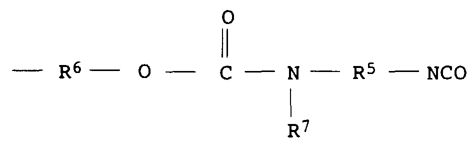 in which
R⁵ is a divalent aliphatic, alicyclic, araliphatic or aromatic C₂-C₂₀ hydrocarbon unit,
R⁶ is a single bond or a divalent aliphatic, alicyclic, araliphatic or aromatic C₁-C₂₀ hydrocarbon unit or a mono- or poly(C₂-C₄-alkylene oxide) unit, and
R⁷ is a carbamoyl radical 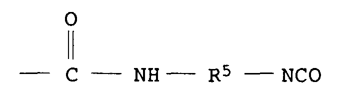 or a biuret group R^{Ib} 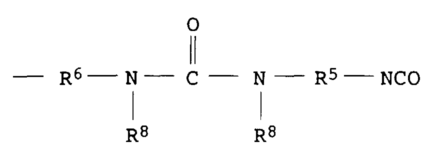 in which one R⁸ is hydrogen and the other is as defined for R⁷
or a biuret group R^{Ic} 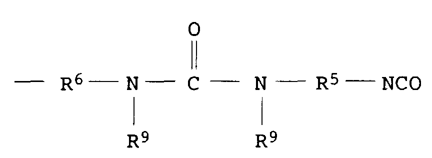 in which one R⁹ is as defined for R⁷ and the other is as defined for R¹
or a thioallophanate group R^{Id}

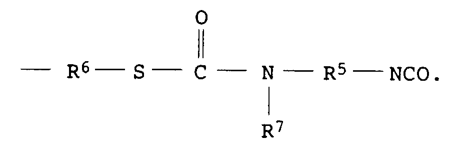

2. A mixture comprising
a) 0.2 - 100 mol% of isocyanate groups in the form of the compound of the general formula I
b) 0 - 99.8 mol% of isocyanate groups in the form of a customary isocyanate having on average at least 2 isocyanate groups (compound II)
c) 0 - 58.2 mol% of isocyanate groups in the form of a compound of the formula III 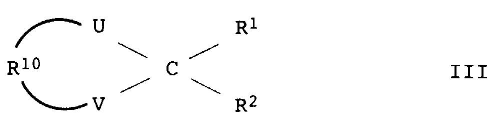 where
R¹ and R² are as defined for formula I,
U and V are -O-, -S- or 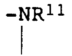 where R¹¹ is hydrogen, C₁-C₁₀-alkyl which is uninterrupted or interrupted by oxygen atoms in ether function, or is C₆-C₁₀-aryl or C₇-C₁₀-aralkyl, and
R¹⁰ is C₁-C₁₀-alkanediyl which together with -U-CR¹R²-V- forms a 4-7-membered ring, in which either one hydrogen in R¹⁰ or the radical R¹¹ in 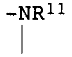 is substituted by a urethane group R^{IIIa} 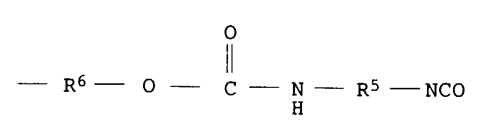 or a urea group R^{IIIb} 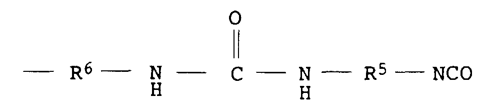 or a urea group R^{IIIc} 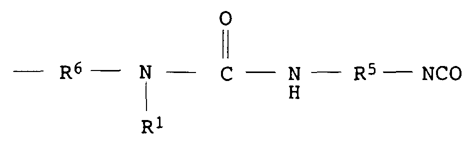 or a thiourethane group R^{IIId} 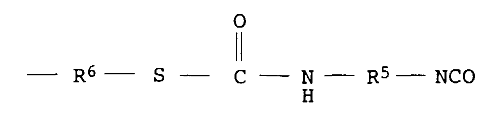 in which R¹, R⁵ and R⁶ are as defined for formula I.

3. A mixture as claimed in claim 2, where the compound II is of the general formula (IIa) 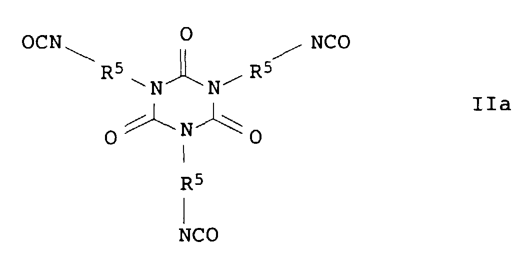 or an oligomeric form deriving therefrom.

4. A process for preparing a mixture as claimed in claim 3, which comprises reacting a compound of the formula IV 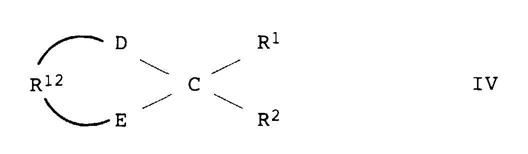 where
R¹ and R² are as defined in formula I, 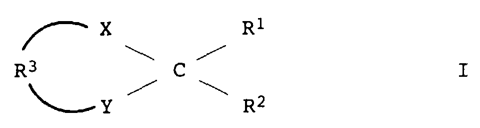
D and E are -O-, -S- or 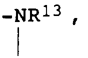 where R¹³ is hydrogen, C₁-C₁₀-alkyl which is uninterrupted or interrupted by oxygen atoms in ether function, or is C₆-C₁₀-aryl or C₇-C₁₀-aralkyl, and
R¹² is C₁-C₁₀-alkanediyl which together with -D-CR¹R²-E- forms a 4-7-membered ring,
in which either one hydrogen in R¹² or the radical R¹³ in 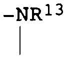 is substituted by
a radical R^{IVa}
-R⁶-OH,
a radical R^{IVb}
-R⁶-NH₂,
or a radical R^{IVc} 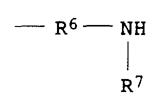 or a radical R^{IVd}
-R⁶-SH
with a compound of the formula V
OCN-R⁵-NCO
at from 20 to 140°C with a molar ratio of the compound of the formula IV to the compound of the formula V of from 1:1.5 to 1:20.

5. A process as claimed in claim 4, wherein the unreacted portions of the compound of the formula V are separated off after the end of the reaction down to a content of less than 1% by weight.

6. A B component for two-component polyurethane coating compositions, comprising
- 0.2 - 99.9 mol% of isocyanate groups in the form of the compound of the formula I
- 0.1 - 99.8 mol% of isocyanate groups in the form of a compound of the formula II and
- 0 - 58.2 mol% of isocyanate groups in the form of a compound of the formula III.

7. A two-component polyurethane coating composition comprising
- a B component as claimed in claim 6 and
- an A component having at least 2 NCO-reactive groups
with the proviso that the molar ratio of the sum formed from the units X, Y, U and V in the compounds of the formulae I and III and the NCO-reactive groups of the A component to the sum of the isocyanate groups in the compounds of the formulae I, II and III is from 0.6:1 to 1.4:1.

8. A one-component polyurethane coating composition comprising
- 0.2 - 100 mol% of isocyanate groups in the form of a compound of the formula I
- 0 - 58.2 mol% of isocyanate groups in the form of a compound of the formula II and
- 0 - 58.2 mol% of isocyanate groups in the form of a compound of the formula III.

9. A coating method which comprises coating an article with a two-component polyurethane coating composition as claimed in claim 6 or 7.

10. A coating method which comprises coating an article with a one-component polyurethane coating composition as claimed in claim 8.

## Revendications

1. Composés comportant des groupements isocyanate et des groupements masqués réactifs vis-à-vis des groupements isocyanate, de formule I 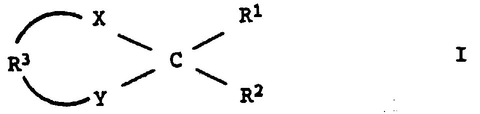 dans laquelle R¹, R², R³, X et Y ont les significations suivantes :
R¹, R² représentent un atome d'hydrogène, un groupement alkyle en C₁-C₁₀, aryle en C₆-C₁₀, aralkyle en C₇-C₁₀ ou bien R¹ et R² forment ensemble un groupement alcanediyle en C₃-C₁₀,
X, Y représentent -O-, -S-, 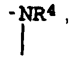 où R⁴ est mis pour un atome d'hydrogène, un groupement alkyle en C₁-C₂₀, pouvant être interrompu par des atomes d'oxygène en fonction éther, un groupement aryle en C₆-C₁₀ ou un groupement aralkyle en C₇-C₁₀,
R³ représente un groupement alcanediyle en C₁-C₁₀, qui forme, avec l'unité -X-CR¹R²-Y- un cycle à 4-7 maillons,
où, soit un des atomes d'hydrogène du motif R³, soit le reste R⁴ de l'unité 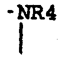 est remplacé par un groupement allophanate R^{Ia} 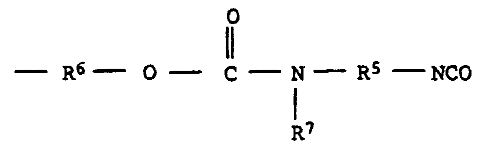 dans lequel
R⁵ représente une unité hydrocarbonée en C₂-C₂₀ divalente, aliphatique, alicyclique, araliphatique ou aromatique et
R⁶ représente une simple liaison ou une unité hydrocarbonée en C₂-C₂₀ divalente, aliphatique, alicyclique, araliphatique ou aromatique ou une unité mono- ou poly-(oxyde d'alkylène en C₂-C₄) et
R⁷ représente un reste carbamoyle 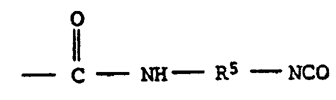 ou un groupement biuret R^{Ib} 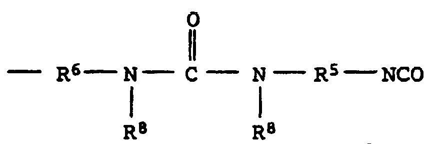 dans lequel l'un des restes R⁸ représente un atome d'hydrogène et l'autre reste prend la même signification que R⁷,
ou un groupement biuret R^{Ic} 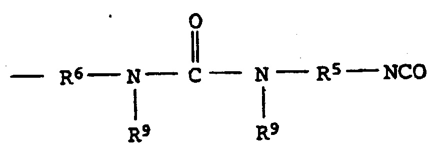 dans lequel l'un des restes R⁹ prend la même signification que R⁷ et l'autre prend la même signification que R¹,
ou un groupement thioallophanate R^{Id}

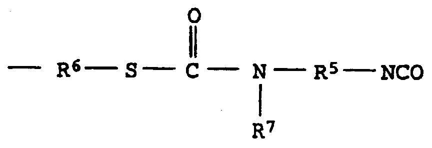

2. Mélanges contenant
a) 0,2-100% en mole de groupements isocyanate sous forme du composé de formule générale I,
b) 0-99,8% en mole de groupements isocyanate sous forme d'un isocyanate usuel ayant en moyenne au moins 2 groupements isocyanate (composé II),
c) 0-58,2% en moles de groupements isocyanate sous forme d'un composé de formule III 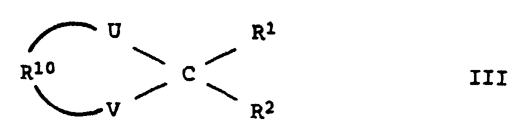 dans laquelle R¹, R², R¹⁰, U et V ont les significations suivantes :
R¹, R² prennent la signification donnée pour la formule I,
U,V représentent -O-, -S-, 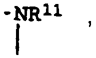 où R¹¹ est mis pour un atome d'hydrogène, un groupement alkyle en C₁-C₁₀, pouvant être interrompu par des atomes d'oxygène en fonction éther, un groupement aryle en C₆-C₁₀ ou un groupement aralkyle en C₇-C₁₀,
R¹⁰ représente un groupement alcanediyle en C₁-C₁₀, qui forme, avec l'unité -U-CR¹R²-V- un cycle à 4-7 maillons,
où, soit un des atomes d'hydrogène du motif R¹⁰, soit le reste R¹¹ de l'unité 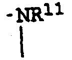 est substitué par un groupement uréthane R^{IIIa} 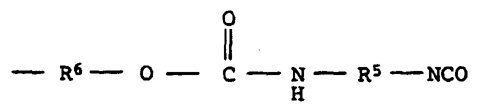 ou un groupement urée R^{IIIb} 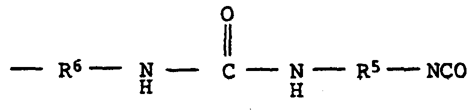 ou un groupement urée R^{IIIc} 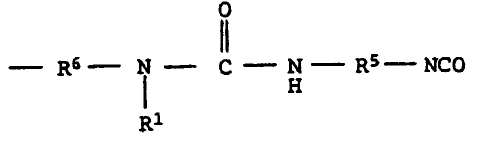 ou un groupement thiouréthane R^{IIId} 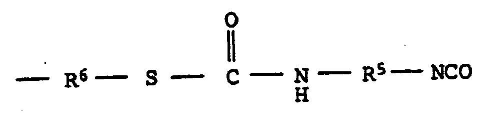 où R¹, R⁵ et R⁶ prennent la signification donnée dans le formule I.

3. Mélanges selon la revendication 2, caractérisés en ce que les composés II sont ceux répondant à la formule générale (IIa) 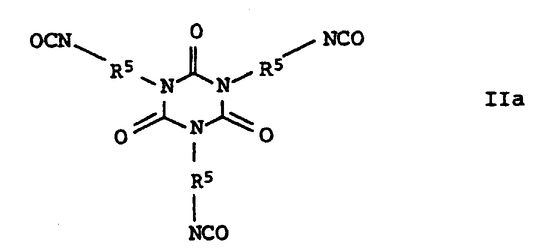 ou les formes oligomères qui en dérivent.

4. Procédé de préparation de mélanges selon la revendication 3, caractérisé en ce que l'on fait réagir un composé de formule IV, 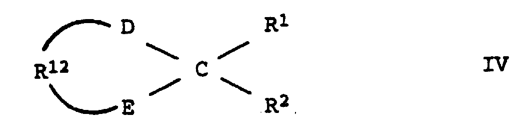 dans laquelle R¹, R², R¹², D et E ont les significations suivantes :
R¹, R² comme pour la formule I, 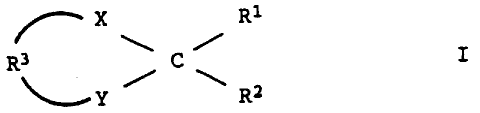
D, E représentent -O-, -S-, 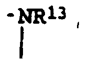 où R¹³ est mis pour un atome d'hydrogène, un groupement alkyle en C₁-C₁₀, pouvant être interrompu par des atomes d'oxygène en fonction éther, un groupement aryle en C₆-C₁₀ ou un groupement aralkyle en C₇-C₁₀,
R¹² représente un groupement alcanediyle en C₁-C₁₃, qui forme, avec l'unité -D-CR¹R²-E- un cycle à 4-7 maillons,
où, soit un des atomes d'hydrogène du motif R¹², soit le reste R¹³ de l'unité 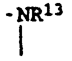 est substitué par un reste R^{IVa}
-R⁶-OH
un reste R^{IVb}
-R⁶-NH₂
ou un reste R^{IVc} 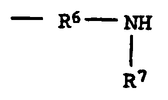 ou un reste R^{IVd}
-R⁶-SH
avec un composé de formule V
OCN-R⁵-NCO
à une température de 20 à 140°C, le rapport en mole du composé de formule IV au composé de formule V s'élevant de 1 : 1,5 à 1 : 20.

5. Procédé selon la revendication 4, caractérisé en ce que, à la fin de la réaction, on sépare la partie du composé de formule V n'ayant pas réagi, jusqu'à une teneur de moins de 1% en poids.

6. Composant B pour masses de revêtement en polyuréthane à deux composants, contenant
- 0,2-99,9% en mole de groupements isocyanate sous forme du composé de formule I,
- 0,1-99,8% en mole de groupements isocyanate sous forme d'un composé de formule II et
- 0-58,2% en moles de groupements isocyanate sous forme d'un composé de formule III.

7. Masses de revêtement en polyuréthane à deux composants, contenant
- un composant B selon la revendication 6 et
- un composant A ayant au moins deux groupements réactifs vis-à-vis de NCO
étant spécifié que le rapport molaire de la somme formée par les unités X, Y, U, V dans les composés de formule I et III ainsi que des groupements du composant A réagissant avec NCO à la somme des groupements isocyanate dans les composés de formule I, II et III s'élève de 0,6 : 1 à 1,4 : 1.

8. Masses de revêtement en polyuréthane à un composant, contenant
- 0,2-100% en mole de groupements isocyanate sous forme du composé de formule I,
- 0-58,2% en mole de groupements isocyanate sous forme d'un composé de formule II et
- 0-58,2% en moles de groupements isocyanate sous forme d'un composé de formule III.

9. Procédé de revêtement, caractérisé en ce que l'on revêt un objet avec une masse de revêtement en polyuréthane à deux composants selon la revendication 6 ou 7.

10. Procédé de revêtement, caractérisé en ce que l'on revêt un objet avec une masse de revêtement en polyuréthane à un composant selon la revendication 8.
